# EUROPEAN PATENT APPLICATION

(11) **EP 3 587 396 A1**
(43) Date of publication of application: **01.01.2020**
(21) Application number: 18180091.3
(22) Date of filing: 27.06.2018
(51) Int. Cl.: C07C 303/40, C07C 205/58

(54) **A METHOD OF ISOLATING A N-(3-NITROBENZOYL)SULFAMIDE**

(71) Applicant: BASF SE, 67056 Ludwigshafen am Rhein (DE)
(72) Inventor: Yerande, Swapnil, 411062 Pune (IN); Kapse, Prashant, 400705 Navia Mumbai (IN); Narayanan, Sukunath, 67056 Ludwigshafen (DE); Goetz, Roland, 67056 Ludwigshafen (DE)
(74) Representative: BASF IP Association

(57) **Abstract**

The presently claimed invention relates to an improved method of isolating 3-nitrobenzenesulfonamide of formula I

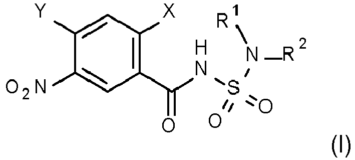

In the 3-nitrobenzenesulfonamide of formula I, R¹ and R² each independently of one another substituted or unsubstituted, linear or branched C₁-C₈-alkyl; substituted or unsubstituted C₃-C₈-cycloalkyl; or R¹ and R² together with the atom to which they are bonded form a 5-, 6-, 7- or 8-membered substituted or unsubstituted, saturated or unsaturated heterocyclic ring which contains 1 or 2 further heteroatoms selected from the group consisting of O and S. The isolation of the 3-nitrobenzenesulfonamide of the formula I from a reaction mixture is performed by acidifying the reaction mixture, separating and washing an organic phase from the acidified reaction mixture to obtain a washed organic phase, obtaining a concentrated organic phase by concentrating a washed organic phase, cooling the concentrated organic phase to obtain a suspension and isolating the 3-nitrobenzenesulfonamide of the formula (I) from the suspension.

## Description

### Field of the invention

The presently claimed invention relates to an improved method of isolating 3-nitrobenzenesulfonamide of formula I 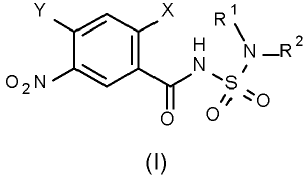 wherein R¹ and R² are each independently selected form the group consisting of substituted or unsubstituted, linear or branched C₁-C₈-alkyl; substituted or unsubstituted C₃-C₈-cycloalkyl; or R¹ and R² together with nitrogen atom to which they are bonded form a 5-, 6-, 7- or 8-membered substituted or unsubstituted, saturated or unsaturated heterocyclic ring which may further contains 1 or 2 heteroatoms selected from O and S; and
X and Y are each independently selected from the group consisting of H, F, Cl, Br, I and CN.

### Background of the Invention

3-Nitrobenzenesulfonamides of formula I are important intermediates for the preparation of active herbicidal ingredients as described in WO 2001/83459 A1. The process for the preparation of nitrobenzenesulfonamides has been described in WO 2004/039768 A1 and WO 2009/050120 A1.

The processes described in the prior arts are notable for obtaining high yield, however, are comparatively complicated owing to the multitude of steps and low purity of the isolated product.

Thus, it is an object of the presently claimed invention to provide a method of isolating 3-nitrobenzenesulfonamide of formula I in high yield with purity above 98%.

### Summary of Invention

Surprisingly, it has been found that 3-nitrobenzenesulfonamide of formula I, can be isolated in high yield with a purity > 98%, from a reaction mixture comprising 3-nitrobenzenesulfonamide of the formula I and other process impurities.

Accordingly, in an aspect, the presently claimed invention is directed to a method of isolating 3-nitrobenzenesulfonamide of formula I from a reaction mixture comprising 3-nitrobenzenesulfonamide of the formula I comprising the steps of:
A) acidifying the reaction mixture with at least one acid to adjust the pH in the range of ≥ 3 to ≤ 6 and obtaining an organic phase and an acidic phase;
B) separating the organic phase and washing the organic phase with at least one base to obtain a washed organic phase and a washed aqueous phase;
C) concentrating the washed organic phase to obtain a concentrated organic phase;
D) cooling the concentrated organic phase to obtain a suspension, and
E) isolating the 3-nitrobenzenesulfonamide of the formula (I) from the suspension.

### Detailed Description of the Invention

Before the present compositions and formulations of the invention are described, it is to be understood that this invention is not limited to particular compositions and formulations described, since such compositions and formulation may, of course, vary. It is also to be understood that the terminology used herein is not intended to be limiting, since the scope of the presently claimed invention will be limited only by the appended claims.

If hereinafter a group is defined to comprise at least a certain number of embodiments, this is meant to also encompass a group which preferably consists of these embodiments only. Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. In case the terms "first", "second", "third" or "(A)", "(B)" and "(C)" or "(a)", "(b)", "(c)", "(d)", "i", "ii" etc. relate to steps of a method or use or assay there is no time or time interval coherence between the steps, that is, the steps may be carried out simultaneously or there may be time intervals of seconds, minutes, hours, days, weeks, months or even years between such steps, unless otherwise indicated in the application as set forth herein above or below.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Furthermore, the ranges defined throughout the specification include the end values as well i.e. a range of 1 to 10 implies that both 1 and 10 are included in the range. For the avoidance of doubt, applicant shall be entitled to any equivalents according to applicable law.

Reference throughout this specification to "one embodiment" or "a preferred embodiment" means that a particular feature, structure or characteristic described in connection with the embodiment is included in at least one embodiment of the presently claimed invention. Thus, appearances of the phrases "in one embodiment" or "in a preferred embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

Accordingly, the presently claimed invention, in one aspect, refers to a method of isolation of 3-nitrobenzenesulfonamide of formula I 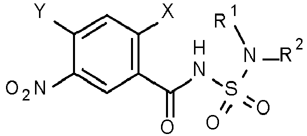 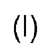 wherein R¹ and R² are each independently selected from the group consisting of substituted or unsubstituted, linear or branched C₁-C₈-alkyl; substituted or unsubstituted C₃-C₈-cycloalkyl; or R¹ and R² together with the nitrogen atom to which they are bonded, form a 5-, 6-, 7- or 8- membered substituted or unsubstituted, saturated or unsaturated heterocyclic ring which, may have further 1 or 2 heteroatoms selected from O and S, and X and Y are each independently selected from the group consisting of H, F, Cl, Br, I and CN,
from a reaction mixture comprising 3-nitrobenzenesulfonamide of the formula I comprising the steps of:
A) acidifying the reaction mixture with at least one acid to adjust the pH in the range of ≥ 3 to ≤ 6 and obtaining an organic phase and an acidic phase;
B) separating the organic phase and washing the organic phase with at least one base to obtain a washed organic phase and a washed aqueous phase;
C) concentrating the washed organic phase to obtain a concentrated organic phase;
D) cooling the concentrated organic phase to obtain a suspension, and
E) isolating the 3-nitrobenzenesulfonamide of the formula (I) from the suspension.

For the purposes of the presently claimed invention, the 3-nitrobenzenesulfonamide of formula I is 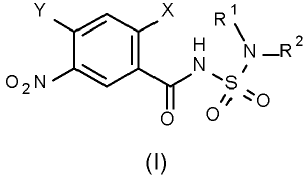 wherein
R¹ and R² are each independently selected from the group consisting of substituted or unsubstituted, linear or branched C₁-C₈-alkyl; substituted or unsubstituted C₃-C₈-cycloalkyl; or R¹ and R² together with the nitrogen atom to which they are bonded, form a 5-, 6-, 7- or 8- membered substituted or unsubstituted, saturated or unsaturated heterocyclic ring which, may have further 1 or 2 heteroatoms selected from O and S, and X and Y are each independently selected form the group consisting of H, F, Cl, Br, I and CN;
preferably, R¹ and R² are each independently selected from the group consisting of substituted or unsubstituted, linear or branched C₁-C₈-alkyl; substituted or unsubstituted C₅-C₇-cycloalkyl; or R¹ and R² together with the nitrogen atom to which they are bonded, form a 5- or 6- membered substituted or unsubstituted, saturated or unsaturated heterocyclic ring which may have further 1 heteroatom selected from O and S, and X and Y are each independently selected form the group consisting of F, Cl, Br, I and CN;
more preferably, R¹ and R² are each independently selected form the group consisting of unsubstituted, linear or branched C₁-C₅-alkyl; unsubstituted C₅-C₇-CyCloalkyl; or R¹ and R² together with the nitrogen atom to which they are bonded, form a 5- or 6- membered unsubstituted, saturated or unsaturated heterocyclic ring which may have further one "O" heteroatom, and X and Y are each independently selected form the group consisting of F, Cl, Br, I;
most preferably, R¹ and R² are each independently of one another unsubstituted, linear or branched C₁-C₅-alkyl and X and Y are each independently of one another F, Cl, Br, I; and
particular preferably, R¹ and R² are each independently of one another linear or branched C₁-C₅-alkyl and X and Y are each independently of one another F and Cl.

Within the context of the presently claimed invention, the term "alkyl", as used herein, refers to an acylic saturated aliphatic groups, including linear or branched alkyl saturated hydrocarbon radical denoted by a general formula CₙH₂ₙ₊₁ and wherein n is the number of carbon atoms 1, 2, 3, 4 etc.

In a preferred embodiment the term "C₁-C₈-alkyl" refers to a substituted or unsubstituted, linear or branched saturated hydrocarbon group having 1 to 8 carbon atoms, more preferably 1 to 5 carbon atoms. The unsubstituted linear C₁-C₈ alkyl which is preferably selected from the group consisting of methyl, ethyl, propyl, butyl, pentyl, hexyl, heptyl and octyl and more preferably, selected from the group consisting of methyl, ethyl, propyl, butyl, pentyl. The unsubstituted branched C₁-C₈ alkyl which is preferably selected from the group consisting, but are not limited to, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, heptyl, octyl and 2-ethylhexyl.

In another preferred embodiment, the term "C₁-C₈-alkyl" refers to a substituted linear or branched saturated hydrocarbon group having 1 to 8 carbon atoms, more preferably 1 to 5 carbon atoms. If one or more of the substituents denote an alkyl residue which is mono- or polysubstituted, this may preferably be substituted with optionally 1, 2, 3, 4 or 5, particularly preferably with 1, 2 or 3, substituents mutually independently selected from the group consisting of F, Cl, Br, I, - NO₂, -CN, -C(=O)-H, -C(=O)-C₁₋₅-alkyl, -C(=O)-phenyl, -C(=S)-C₁₋₅-alkyl, -C(=S)-phenyl, -C(=O)-OH, -C(=O)-O-C₁₋₅-alkyl, -C(=O)-O-phenyl, -C(=O)-NH₂, -C(=O)-NH-C₁₋₅-alkyl, -C(=O)-N(C₁₋₅-alkyl)₂, -S(=O)-C₁₋₅-alkyl, -S(=O)-phenyl, -S(=O)₂-C₁₋₅-alkyl, -S(=O)₂-phenyl, -S(=O)₂-NH₂ and - SO₃H, wherein the above-stated-C₁₋₅ alkyl residues may in each case be linear or branched and the above-stated phenyl residues may preferably be substituted with 1, 2, 3, 4 or 5 substituents mutually independently selected from the group consisting of F, Cl, Br, I, -CN, -CF₃, -OH, -O-CF₃, -SH, -O-CH₃, -O-C₂H₅, -O-C₃H₇, methyl, ethyl, n-propyl, isopropyl, n-butyl, 2-butyl, isobutyl and tert-butyl. Particularly preferred substituents may be selected mutually independently from the group consisting of F, Cl, Br, I, -NO₂, -CN, -OH and -SH.

For the purpose of the presently claimed invention, the term "C₃-C₈-cycloalkyl" refers to a monocyclic and bicyclic 3 to 8 membered saturated cycloaliphatic radical.

In a preferred embodiment, R₁ is substituted or unsubstituted C₃-C₈ cycloalkyl is monocyclic and bicyclic preferably selected from C₃-C₆, more preferably C₅-C₆ cycloalkyl. Representative examples of unsubstituted or branched C₃-C₈ monocyclic and bicyclic cycloalkyl include, but are not limited to, cyclopropyl, cyclobutyl, cyclopentyl, cyclohexyl, cycloheptyl, bicyclo[2.2.1]heptyl and bicyclo[3.1.1]heptyl.

In a preferred embodiment, substituted C₃-C₈cycloalkyl means a cycloalkyl residue having one or more substituents. The substituents are preferably selected from the group consisting of oxo (=O), F, Cl, Br, I, -NO₂, -CN, , -C(=O)-C₁₋₅-alkyl, -C(=O)-phenyl, -C(=S)-C₁₋₅-alkyl, -C(=S)-phenyl, -C(=O)-O-C₁₋₅-alkyl, -C(=O)-O-phenyl, -C(=O)-NH₂, -C(=O)-NH-C₁₋₅-alkyl, -C(=O)-N(C₁₋₅-alkyl)₂, - S(=O)-C₁₋₅-alkyl, -S(=O)-phenyl, -S(=O)₂-C₁₋₅-alkyl, -S(=O)₂-phenyl and -S(=O)₂-NH₂, wherein the above-stated-C₁₋₅ alkyl residues may in each case be linear or branched and the above-stated phenyl residues may preferably be substituted with 1, 2, 3, 4 or 5 substituents mutually independently selected from the group consisting of F, Cl, Br, I, -CN, -CF₃, -O-CF₃, -O-CH₃, -O-C₂H₅, -O-C₃H₇, methyl, ethyl, n-propyl, isopropyl, n-butyl, 2-butyl, isobutyl and tert-butyl. Particularly preferred substituents may be selected mutually independently from the group consisting of oxo (=O), F, Cl, Br, I, -NO₂ and -CN.

In a preferred embodiment R¹ and R² together with the nitrogen atom to which they are bonded, form a 5-, 6-, 7- or 8-membered substituted or unsubstituted, saturated or unsaturated heterocyclic ring which may further contains 1 or 2 heteroatoms selected from O and S.
Examples of 5-, 6-, 7- or 8-membered saturated or unsaturated heterocyclic ring which contains 1 or 2 further heteroatoms selected from the group consisting of O and S are, but not limited to, pyrrolidin-1-yl, imidazol-1-yl, oxazolidin-3-yl, isoxazolidin-2-yl, thiazolidin-3-yl,isothiazolidin-2-yl, ,piperidin-1-yl, morpholin-4-yl, thiomorpholin-4-yl, 1-oxo-1,4-thiazinan-4-yl, 1,1-dioxothiazinan-2-yl 2,3-dihydropyrrol-1-yl, 2,5-dihydropyrrol-1-yl, ,3,4-dihydro-2H-pyridin-1-yl, 3,6-dihydro-2H-pyridin-1-yl, 1-piperidyl, 2-methylmorpholin-4-yl, 3-methylmorpholin-4-yl, 2,3-dimethylmorpholin-4-yl, 2,3-dimethylmorpholin-4-yl, 3-methyl-1-piperidyl and 2,3-dimethyl-1-piperidyl.
In another preferred embodiment, X and Y are each independently selected from the group consisting of H, F, Cl, Br, I and CN; more preferably X and Y are each independently selected from the group consisting of H, F, Cl, Br and I; and most preferably from each independently selected from F and Cl.

In another preferred embodiment, the reaction mixture is prepared by reacting a sulfuric diamide of the formula (i)

R¹R²N-S(O)₂-NH₂ (i)

with a 3-nitrobenzoyl chloride of a formula (ii)

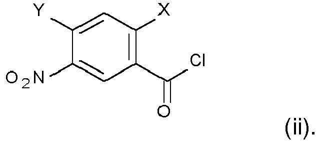

In this context, the variables R¹, R², X and Y used in formulae (i) and (ii) are each as defined as given above.

The process of preparing the reaction mixture is known in principle from the prior art. The reaction of the sulfuric diamide of the formula (i) with a nitrobenzoyl chloride of the formula (ii) can be carried out, for example, according to scheme 2 on page 15 of WO 2004/039768 A1, and according to the information on pages 16 to 19 or example 1 on page 56 of WO 2004/039768 A1 or according to the examples adduced here.

In a preferred embodiment, the reaction mixture is prepared by
(a) mixing the sulfuric diamide of the formula (i) with at least one aromatic solvent and a phase transfer catalyst; to obtain a mixed solution, and
(b) adding a base solution and 3-nitrobenzoyl chloride of the formula (ii) to the mixed solution of step (a) to form the reaction mixture.

Aromatic solvents are solvents whose main constituents are aromatic compounds which are derived from benzene and are liquid at room temperature. Such aromatic compounds include, for example, benzene, alkylbenzenes such as toluene, xylenes, trimethylbenzene and ethylbenzene, and also chlorinated and/or fluorinated benzenes such as chlorobenzene, fluorobenzene and dichlorobenzenes.

The preferred aromatic compounds which are useful as the at least one aromatic solvent is benzene, alkylbenzenes such as toluene, xylenes, trimethylbenzene and ethylbenzene, and also chlorinated and/or fluorinated benzenes such as chlorobenzene, fluorobenzene and dichlorobenzenes. In a more preferred embodiment, the at least one aromatic solvent comprises chlorobenzene. Even more preferably, the at least one aromatic solvent comprises at least 80%, yet more preferably at least 90 % or at least 95% by volume chlorobenzene. In particular, the at least one aromatic solvent comprises at least 95% and especially at least 98% by volume chlorobenzene or the at least one aromatic solvent consists of chlorobenzene, in each case based on the total amount of solvent.

In addition to these aromatic solvent, the at least one aromatic solvent may also comprise up to 50% by volume, preferably not more than 30% by volume and more preferably not more than 10% by volume of different solvents. The example for different solvents other than aromatic solvents are aliphatic halohydrocarbons, aliphatic or cycloaliphatic hydrocarbons. The examples for halohydrocarbons are, but not limited to, dichloromethane, trichloromethane and/or dichloroethane. The examples for aliphatic or cycloaliphatic hydrocarbons are, but not limited to, hexane, heptane, octane, cyclohexane, cycloheptane, cyclooctane and mixtures thereof. In addition, the at least one aromatic solvent may also comprise small amounts of non-inert, aprotic oxygen-containing and/or nitrogen-containing solvents, in which case their proportion makes up preferably not more than 10% by volume, based on the total amount of the aromatic solvent. The at least one aromatic solvent preferably comprises no oxygen-containing and/or nitrogen-containing, aprotic solvents (<1 % by volume).

In a preferred embodiment, the reaction mixture contains at least one phase transfer catalysts. The suitable phase transfer catalysts which may be mentioned, but not limited to, are the following: tetraalkyl-(C₁-C₁₈)-ammonium halide, N-benzyl trialkyl-(C₁-C₁₈)-ammonium halide, tetraalkyl-(C₁-C₁₈)-phosphonium halides, tetraphenylphosphonium halide, (phenyl)ₒ(C₁-C₁₈-alkyl)ₚ-phosphonium halide, wherein o=1 to 3, p=3 to 1 and always o+p =4; and halogen is selected from fluoride, chloride, bromide and iodide, more preferably chloride and bromide.

In a preferred embodiment, the phase transfer catalysts are, but not limited to, tetraethylammonium chloride, N-benzyltriethylammonium chloride or tributylmethylammonium chloride hereinafter referred as Bu₃MeNCl is used. The amount of phase-transfer catalyst is generally up to 20% by weight, preferably from 1 to 15% by weight and particularly preferably from 2 to 8% by weight, based on the sulfuric diamide of the formula (i). In a preferred embodiment, the phase transfer catalyst used is Bu₃MeNCl.

In a preferred embodiment, in step (a) the mixed solution is prepared by mixing the sulfuric diamide of the formula (i) with chlorobenzene and Bu₃MeNCl to obtain the mixed solution.

In a preferred embodiment, the sulfuric diamide of formula (i) is 2-[methyl(sulfamoyl)amino]propane (ia).

In a preferred embodiment, the mixed solution obtained in step (a) is cooled at a temperature in the range of ≥ 0°C to ≤ 30°C, more preferably at a temperature in the range of ≥ 0°C to ≤ 25°C, or ≥ 0°C to ≤ 20°C, or ≥ 0°C to ≤ 15°C, or ≥ 5°C to ≤ 15°C. In the most preferred embodiment, the mixed solution in step (a) is cooled at a temperature in the range of ≥ 10°C to ≤ 15°C.

In a preferred embodiment, a base solution is added to the mixed solution obtained in step (a). The base is selected from alkali metal and alkaline earth metal hydroxides, more preferably selected from the group consisting of lithium hydroxide, sodium hydroxide, magnesium hydroxide, potassium hydroxide, and calcium hydroxide, most preferably selected from the group consisting of sodium hydrooxide and potassium hydroxide.

In a preferred embodiment, the base solution is prepared with NaOH and water; more preferably, the NaOH solution has 50% w/v in water.

In another preferred embodiment, 50 % NaOH solution is added to a mixed solution of step a). The addition of NaOH solution was completed within 60 minutes. After 15 minutes of start of addition of NaOH solution, initiated simultaneous addition of 3-nitrobenzoyl chloride of formula (ii) 50% w/w solution in chlorobenzene to the mixed solution to obtain the reaction mixture.

In a preferred embodiment, the 3-nitrobenzoyl chloride of formula (ii) is 2-chloro-4-fluoro-5-nitrobenzoyl chloride(iia).

In a preferred embodiment, the 3-nitrobenzenesulfonamide of formula (I) in the reaction mixture is 2-chloro-4-fluoro-N-[isopropyl(methyl)sulfamoyl]-5-nitro-benzamide(la).

In a preferred embodiment, the reaction mixture is obtained by mixing 2-[methyl(sulfamoyl)amino]propane (ia) with chlorobenzene and Bu₃MeNCl followed by addition of NaOH solution and 2-chloro-4-fluoro-5-nitro-benzoyl chloride (iia) in chlorobenzene. The progress of the reaction is monitored by UPLC. Upon completion of reaction the product was isolated from the reaction mixture using following steps.

### Step A)

In an embodiment, in step A) the reaction mixture containing 3-nitrobenzenesulfonamide of formula I and other process impurities, is acidified with at least one acid to obtain an organic phase and an aqueous phase.

In a preferred embodiment the at least one acid is selected from the group consisting of an inorganic acid and an organic acid. Preferably the inorganic acid is selected from the group consisting of hydrochloric acid, nitric acid, phosphoric acid, sulphuric acid, boric acid, hydrofluoric acid, hydrobromic acid, phosphoric acid and perchloric acid. More preferably the inorganic acid is selected from the group consisting of hydrochloric acid and sulfuric acid, most preferably the inorganic acid is sulfuric acid.

In a preferred embodiment the organic acid is selected from a carboxylic acid and a sulfonic acid. The carboxylic acid is selected from the group consisting of lactic acid, acetic acid, formic acid, citric acid, oxalic acid, uric acid and malic acid. More preferably the carboxylic acid is selected from acetic acid and formic acid. The sulfonic acid is selected from the group consisting of methane sulfonic acid, trifluoromethane sulfonic acid, trichloromethane sulfonic acid, p-toluene sulfonic acid, and more preferably the sulfonic acid is methane sulfonic acid.

In a preferred embodiment the pH of the reaction mixture is adjusted to a value in the range of ≥ 3.0 to ≤ 6.0. More preferably the pH is adjusted to a value in the range of ≥ 3.0 to ≤ 5.7, or ≥ 3.0 to ≤ 5.5, or ≥ 3.0 to ≤ 5.25, or ≥ 3.0 to ≤ 5, or ≥ 3.0 to ≤ 4.7, or ≥ 3 to ≤ 4.5, or ≥ 3.0 to ≤ 4.25. Most preferably the pH is adjusted to a value between ≥ 3.0 to ≤ 4.0, particular preferably the pH of the reaction mixture is adjusted to a value in the range of 3 to 4.

In a preferred embodiment the reaction mixture is acidified with at least one acid in step A) while the temperature is maintained in the range of ≥ 50°C to ≤ 80°C, more preferably at a temperature in the range of ≥ 50°C to ≤ 75°C, or ≥ 55°C to ≤ 75°C, or ≥ 60°C to ≤ 75°C even more preferably at a temperature in the range of ≥ 65°C to ≤ 75°C for a period of ≥ 5 to ≤ 30 min. In the most preferred embodiment, the reaction mixture is acidified with at least one acid in step A) at 70°C, over a period of 15 min.

In a preferred embodiment, the at least one acid in step A) is added completely or in several portions to the reaction mixture until the desired pH value is achieved.

In another preferred embodiment, the at least one acid is present in the form of an aqueous solution.

In the most preferred embodiment, the reaction mixture in step A) is added completely or in several portions to the at least one acid. The pH is then adjusted to the desired value. The addition is performed by any suitable methods known to the person skilled in the art with or without using suitable dosing equipment.

In another preferred embodiment, the at least one acid is added to the reaction mixture of step A) completely or in several portions.

In a preferred embodiment, the addition of the at least one acid to the reaction mixture in step A) is performed by slow manual pouring, or dosing by suitable dosing equipment such as, but not limited to a peristaltic pump and a diaphragm pump (hydraulic actuated, air operated devices, etc.).

### Step B)

In step B), the organic phase and the acidic phase obtained in step A) are separated from each other. Then the organic phase is washed with at least one base to obtain a washed organic phase and a washed aqueous phase.

In a preferred embodiment the at least one base is selected from the group consisting of sodium acetate, lithium acetate, potassium acetate, sodium carbonate, and potassium carbonate. More preferably, the at least one base is sodium acetate and present in the form of an aqueous solution.

In a preferred embodiment, the organic phase is washed with at least one base is by stirring at a temperature in range of ≥ 50°C to ≤ 90°C. More preferably the stirring is performed at a temperature in the range of ≥ 50°C to ≤ 85°C, or ≥ 55°C to ≤ 85°C, or ≥ 60°C to ≤ 85°C, or ≥ 60°C to ≤ 80°C, or ≥ 65°C to ≤ 80°C. Stirring refers to mechanical stirring using any suitable stirrer known to a person skilled in the art. The method of stirring in general is not limited by the type and rotation speed of the stirrer employed therein. These parameters are well known in the art.

In another preferred embodiment, stirring in step B) is performed for a time period of ≥ 5 min to ≤ 30 min.

In another preferred embodiment, the organic phase that is washed with the at least one base is allowed to settle to obtain the washed organic phase and the washed aqueous phase after washing.

In another preferred embodiment, the washed organic phase obtained in step B) is separated from the washed aqueous phase.

The separation process divides two distinct or immiscible or wetting/ non-wetting phases. The separation is a process well-known to the person skilled in the art and includes, but is not limited to, single stage extraction, gravity separation, membrane separators, etc. In a preferred embodiment, the separation is performed by using a separating funnel. In another preferred embodiment, the separation is performed by using a mixer settler.

In yet another preferred embodiment, the separation is performed by using a membrane separator such as but not limited to a liquid-liquid separator.

### Step C)

In an embodiment, in step C), the washed organic phase obtained in step B) is concentrated. Preferably, the washed organic phase is concentrated by atmospheric distillation or vacuum distillation.

In another preferred embodiment, the organic phase is concentrated to 50 to 70% w/w, more preferably 50 to 60 % w/w and most preferably 50 to 55 % w/w of total w/w % of the product and solvent as determined by UPLC.

In another preferred embodiment, the concentrated organic phase obtained in step C) is present in a liquid form or in a solid form.

In another embodiment, the step C optionally comprises at least one sub-step selected from the group consisting of C1 to C5 as defined herein.
C1) heating the concentrated organic phase of step C) to a temperature in the range of ≥ 50°C to ≤ 70°C for a period of ≥ 5 to ≤ 60 min,
C2) adding water to the concentrated organic phase of step C), followed by heating to a temperature in the range of ≥ 60°C to ≤ 90°C, and concentrating,
C3) adding at least one alcohol to the concentrated organic phase of step C), heating to a temperature in the range of ≥ 50°C to ≤ 90°C, followed by adding water, and heating to a temperature in the range of ≥ 70°C to ≤ 90°C,
C4) heating the concentrated organic phase to a temperature in the range of ≥ 70°C to ≤ 90°C, adding at least one hydrophobic solvent to the concentrated organic phase of step C) and maintaining the temperature in the range of ≥ 70°C to ≤ 90°C for a period of ≥ 5 to ≤ 120 min, and
C5) adding water to the concentrated organic phase of step C), heating to a temperature in the range of ≥ 60°C to ≤ 90°C, concentrating, and adding at least one alcohol.

In a preferred embodiment, the at least one alcohol in the sub-steps C1) to C5) is selected from the group consisting of methanol, ethanol, n-propanol, isopropanol, n-butanol, tert-butanol, and aqueous solution thereof. More preferably the alcohol is methanol or its aqueous solution.

In a preferred embodiment, the at least one hydrophobic solvent in the sub-steps C1) to C5) is selected from the group consisting of pentane, hexane, cyclopentane, cyclohexane, toluene, xylene, chlorinated hydrocarbons such as methylene chloride, chloroform, 1,2-dichloroethane, 1,1,2,2-tetracholrethane, chlorobenzene and 1,2-, 1,3-, or 1,4-dichlorobenzene.

### Step D)

In an embodiment, in step D), the concentrated organic phase obtained in step C) is cooled to obtain a suspension.

In a preferred embodiment, the concentrated organic phase in step D) is cooled to a temperature in the range of ≥ 0°C to ≤ 35°C, more preferably the concentrated organic phase is cooled to a temperature in range of ≥ 0°C to ≤ 5°C, most preferably 0 to 10 °C and particularly preferably 0 to 5°C.

In yet another more preferred embodiment, the concentrated organic phase is cooled at a temperature in range of ≥ 25°C to ≤ 35°C, more preferably the concentrated organic phase is cooled at a temperature in range of ≥ 30°C to ≤ 35°C.

### Step E)

In step E), the 3-nitrobenzenesulfonamide of formula (I) is isolated from the suspension.

In a preferred embodiment, in step E), the 3-nitrobenzenesulfonamide of the formula (I) is isolated from the concentrated organic phase by filtration.

In another preferred embodiment, in step E) the 3-nitrobenzenesulfonamide of the formula (I) is isolated from the concentrated washed organic phase by filtration and drying, or a combination thereof with washing.

In a more preferred embodiment, the suspension is filtered and dried.

In yet another more preferred embodiment, the drying is performed under vacuum.

In yet another more preferred embodiment, the drying is performed at a temperature in range of 40°C to 75°C, more preferably in the range of 50 to 70°C.

In the most preferred embodiment, the suspension of step D) is filtered, washed and then dried.

The washing is performed with at least hydrophobic solvent is selected from the group consisting of pentane, hexane, cyclopentane, cyclohexane, toluene, xylene, chlorinated hydrocarbons such as methylene chloride, chloroform, 1,2-dichloroethane, 1,1,2,2-Te-tracholrethane, chlorobenzene and 1,2-, 1,3-, or 1,4-dichlorobenzene. Most preferably, the suspension is filtered washed with 1:1 mixture of n-hexane and chlorobenzene.

### Industrial Application/ Advantages

The method according to the presently claimed invention is associated with a series of advantages. Firstly, the method according to the presently claimed invention is comparatively easy to perform. The method can additionally be handled efficiently on the industrial scale. The method facilitates in obtaining high yield of the 3-nitrobenzenesulfonamide with high purity. The method reduces extent of industrial waste generated in aqueous and organic phases/ streams.

In the following, specific embodiments of the presently claimed invention are described:
1. A method of isolating a 3-nitrobenzenesulfonamide of the formula (I), 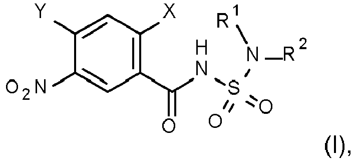 wherein
   R¹ and R² are each independently selected from the group consisting of substituted or unsubstituted, linear or branched C₁-C₈-alkyl; substituted or unsubstituted C₃-C₈-cycloalkyl; or R¹ and R² together with the nitrogen atom to which they are bonded, form a 5-, 6-, 7- or 8-membered substituted or unsubstituted, saturated or unsaturated heterocyclic ring which, may have further 1 or 2 heteroatoms selected from O and S, and
   X and Y are each independently selected from the group consisting of H, F, Cl, Br, I and CN,
   from a reaction mixture comprising the 3-nitrobenzenesulfonamide of the formula I comprising at least the steps of
   A) acidifying the reaction mixture with at least one acid to adjust the pH in the range of ≥ 3 to ≤ 6 and obtaining an organic phase and an aqueous phase;
   B) separating the organic phase and washing the organic phase with at least one base to obtain a washed organic phase and a washed aqueous phase;
   C) concentrating the washed organic phase obtained in step B);
   D) cooling the concentrated organic phase to obtain a suspension, and
   E) isolating the 3-nitrobenzenesulfonamide of the formula (I) from the suspension.
2. The method according to embodiment 1, wherein
   R¹ is methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl or 1-ethylpropyl;
   R² is methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl or 1-ethylpropyl;
   X is F or Cl; and
   Y is F or Cl.
3. The method according to embodiment 1, wherein the reaction mixture is obtained by reacting a sulfuric diamide of the formula (i)

   R¹R²N-S(O)₂-NH₂ (i)

   in which R¹ and R² are each defined as in embodiment 1 or 2,
   with a 3-nitrobenzoyl chloride of the formula (ii) 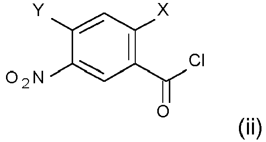 in which X and Y are each defined as in embodiment 1 or 2.
4. The method according to embodiment 3, wherein the reaction mixture is obtained by
   (a) mixing the sulfuric diamide of the formula (i) with at least one aromatic solvent and at least one phase transfer catalyst; to obtain a mixed solution, and
   (b) adding a base solution and 3-nitrobenzoyl chloride of the formula (ii) to the mixed solution of step (a) to form the reaction mixture.
5. The method according to embodiment 4, wherein the at least one aromatic solvent is selected from the group consisting of benzene, alkylbenzenes and halobenzene.
6. The method according to embodiment 5, wherein the alkylbenzenes are selected from the group consisting of toluene, xylenes, trimethylbenzene, and ethylbenzene.
7. The method according to embodiment 5, wherein the halobenzenes are selected from the group consisting of chlorobenzene, fluorobenzene, and dichlorobenzenes.
8. The method according to embodiment 4, wherein the at least one phase transfer catalyst is selected from the group consisting of tetraalkyl-(C₁-C₁₈)-ammonium halide, N-benzyl trialkyl-(C₁-C₁₈)-ammonium halide, tetraalkyl-(C₁-C₁₈)-phosphonium halides, tetraphenylphosphonium halide, (phenyl)ₒ(C₁-C₁₈-alkyl)ₚ-phosphonium halide, wherein o=1 to 3, p=3 to 1 and always o+p =4; and halogen is selected from fluoride, chloride, bromide and iodide.
9. The method according to embodiment 4, wherein the at least one phase transfer catalyst is selected from the group consisting of tetraethylammonium chloride, N-benzyltriethylammonium chloride, and tributylmethylammonium chloride.
10. The method according to embodiment 4, wherein the temperature in step (a) is in the range of ≥ 10 °C to ≤ 15°C.
11. The method according to embodiment 4, wherein the base is selected from the group consisting of alkali metal and alkaline earth metal hydroxides.
12. The method according to embodiment 11, wherein the alkali metal and alkaline earth metal hydroxide are selected from the group consisting of lithium hydroxide, sodium hydroxide, magnesium hydroxide, potassium hydroxide, and calcium hydroxide.
13. The method according to embodiment 4, wherein the sulfuric diamide of the formula (i) is 2-[methyl(sulfamoyl)amino]propane.
14. The method according to embodiment 4, wherein the 3-nitrobenzoyl chloride of the formula (ii) is 2-chloro-4-fluoro-5-nitro-benzoyl chloride.
15. The method according to embodiment 4, wherein the 3-nitrobenzenesulfonamide of formula (I) is 2-chloro-4-fluoro-N-[isopropyl(methyl)sulfamoyl]-5-nitro-benzamide.
16. The method according to embodiment 1, wherein in step A) the at least one acid is selected from inorganic acid and organic acid.
17. The method according to embodiment 16, wherein the inorganic acid is selected from the group consisting of hydrochloric acid, nitric acid, phosphoric acid, sulphuric acid, boric acid, hydrofluoric acid, hydrobromic acid; and perchloric acid.
18. The method according to embodiment 16, wherein the organic acid is selected from carboxylic acids and sulfonic acids.
19. The method according to embodiment 18, wherein the carboxylic acids is selected from the group consisting of lactic acid, acetic acid, formic acid, citric acid, oxalic acid, uric acid and malic acid.
20. The method according to embodiment 18, wherein the sulfonic acids is selected from the group consisting of methane sulfonic acid, trifluoromethane sulfonic acid, trichloromethane sulfonic acid, p-toluene sulfonic acid
21. The method according to embodiment 1, wherein the pH of step A) is in the range of ≥ 3 to ≤ 4.
22. The method according to embodiment 1, wherein the reaction mixture of step A) is maintained at a temperature in the range of 50°C to 80°C for a period of 5 to 30 min.
23. The method according to embodiment 1, wherein the at least one base in step B) is selected from the group consisting of sodium acetate, lithium acetate, potassium acetate. sodium carbonate and potassium carbonate.
24. The method according to embodiment 1, wherein in step B) the organic phase is washed by stirring organic phase with the at least one base at a temperature in the range of ≥ 50°C to ≤ 90°C.
25. The method according to embodiment 1, wherein in step C) the washed organic phase is concentrated by a method selected from the group consisting of distillation, vacuum distillation, osmosis, reverse osmosis and the like.
26. The method according to embodiment 1, wherein the concentrated organic phase of step C) is treated by at least one-sub-step selected from the steps of:
   C1) heating the concentrated organic phase of step C) to a temperature in the range of ≥ 50°C to ≤ 70°C for a period of ≥ 5 to ≤ 60 min,
   C2) adding water to the concentrated organic phase of step C), heating to a temperature in the range of ≥ 60°C to ≤ 90°C, and concentrating,
   C3) adding at least one alcohol to the concentrated organic phase of step C), heating to a temperature in the range of ≥ 50°C to ≤ 90°C, adding water, and heating to a temperature in the range of ≥ 70°C to ≤ 90°C,
   C4) heating the concentrated organic phase to a temperature in the range of ≥ 70°C to ≤ 90°C, adding at least one hydrophobic solvent to the concentrated organic phase of step C) and maintaining at a temperature in the range of ≥ 70°C to ≤ 90°C for a period of ≥ 5 to ≤ 120 min, and
   C5) adding water to the concentrated organic phase of step C), heating to a temperature in the range of ≥ 60°C to ≤ 90°C, concentrating, and adding at least one alcohol.
27. The method according to embodiment 26, wherein the alcohol is selected from the group consisting of methanol, ethanol, n-propanol, isopropanol, n-butanol, and aqueous solution thereof.
28. The method according to embodiment 26, wherein the hydrophobic solvent is selected from the group consisting of pentane, hexane, cyclopentane, cyclohexane, toluene, xylene, methylene chloride, chloroform, 1,2-dichloroethane, 1,1,2,2-Tetracholrethane, chlorobenzene and 1,2-, 1,3-, or 1,4-dichlorobenzene.
29. The method according to embodiment 1, wherein in step D) the concentrated organic phase is cooled to a temperature in the range of ≥ -5°C to ≤ 35°C.
30. The method according to embodiment 1, wherein in step E) the 3-nitrobenzenesulfonamide of the formula (I) is isolated by filtering the suspension.
31. The method according to embodiment 30, wherein in step E) the filtering of the suspension is followed by drying.
32. The method according to embodiment 31, wherein the 3-nitrobenzenesulfonamide of the formula (I) is dried at a temperature in the range of 40°C to 75°C.
33. The method according to embodiment 30, wherein the filtration of the suspension in step E) is followed by washing before drying.
34. The method according to embodiment 33, wherein the washing in step E) is performed with water or at least one hydrophobic solvent selected from the group consisting of pentane, hexane, cyclopentane, cyclohexane, toluene, xylene, chlorinated hydrocarbons such as methylene chloride, chloroform, 1,2-dichloroethane, 1,1,2,2-Tetracholrethane, chlorobenzene and 1,2-, 1,3-, or 1,4-dichlorobenzene.

### Examples

The presently claimed invention is further illustrated in combination with the following examples. These examples are provided to exemplify the presently claimed invention, but are not intended to restrict the scope of the presently claimed invention in any way. The terms and abbreviations in the examples have their common meanings. For example, "°C", "HPLC", "UPLC", "% w/w", "% w/v" and "gm" represent "degree Celsius", "high performance liquid chromatography", "ultra-Performance Liquid chromatography", "percent weight by weight", "percent weight by volume" and "gram" respectively.

### Example 1

2-[methyl(sulfamoyl)amino]propane (0.39 moles, 62.35 gm), chlorobenzene (12 volumes, 870 gm) and Bu₃MeNCl(1.29 gm) were charged to a reaction flask to obtain a mixed solution. The mixed solution was cooled to a temperature of 15°C and charged 50% aq. NaOH solution (55gm) to the above mixture over a period of 60 min. After 15 min of starting the addition of the NaOH solution, charged a solution of 2-chloro-4-fluoro-5-nitro-benzoyl chloride (0.34 moles) in chlorobenzene (50% w/w) to the mixed solution over a period of 45 minutes. The progress of the reaction was monitored by UPLC.

In step A), after completion of the reaction, the reaction was quenched by pouring into aqueous H₂SO₄ solution (18 gm 98% H₂SO₄ make up to 340 gm) by maintaining a temperature below 80°C. The pH of the mixture was adjusted in range of 3 to 4, and stirred at a temperature in the range of 65 to 80°C for 15 to 20 min and allowed to settle. The organic phase was separated from the acidic phase.

In step B), the organic phase obtained in step A) was washed with aq. sodium acetate solution (2.5 gm NaOAc diluted to 180 gm by water), stirred at temperature of 75°C for 15 min, and allowed to settle. Then, the washed organic phase and the washed aqueous phase was separated.

In step C), the washed organic phase obtained in step B) was concentrated to obtain 52.6 % solution of the concentrated organic phase having 2-chloro-4-fluoro-N-[isopropyl(methyl)sulfamoyl]-5-nitro-benzamide (Ia) with UPLC purity of 97.6 %.

In sub-step C1) the concentrated organic phase of the product was heated to a temperature in range of 60°C to 65°C and maintained for 30 min.

In step D), the concentrated organic phase was gradually cooled to a temperature in range of 0°C to 5°C to obtain a suspension.

In step E), the suspension obtained was filtered and dried under vacuum at a temperature of 70°C to obtain the product 2-chloro-4-fluoro-N-[isopropyl(methyl)sulfamoyl]-5-nitro-benzamide(la) (105.9 gm, 90.2%) with the UPLC purity of 99.37 %, (101.16 % w/w).

### Example 2

In example 2, the reaction was performed similar to example 1 until step B).

In step B), the organic phase obtained in step A) was washed with aq. NaOAc solution (1.8 gm NaOAc diluted to 180 gm by water), at temperature of 75°C for 15 min and allowed to settle. The settled organic phase and aqueous phase was separated to obtain the washed organic phase and the washed aqueous phase.

In step C), the washed organic phase obtained in step B) was concentrated to obtain a solution having 50 % of the product, 2-chloro-4-fluoro-N-[isopropyl(methyl)sulfamoyl]-5-nitro-benzamide (Ia) with the UPLC purity of 98.19 %.

In sub-step C2), 300 mL water was added to the concentrated organic phase and heated to a temperature in the range of 80°C to 85°C. The chlorobenzene was removed by vacuum distillation and maintained the quantity of 300 mL water having pH 3.1. The pH of the solution was adjusted using H₂SO₄, if required. After complete removal of chlorobenzene, the solution was maintained for 30 min at a temperature of 80°C to 85°C.

In step D), the concentrated organic phase was gradually cooled to a temperature of 25°C to 30°C to obtain a suspension.

In step E), the suspension obtained was filtered, washed with 50 mL water and then dried under vacuum at a temperature of 70°C to obtain the product, 2-chloro-4-fluoro-N-[isopropyl(methyl)sulfamoyl]-5-nitro-benzamide(la) (107 gm, 91.1 %) with UPLC purity of 99.13 %, (98.72 % w/w).

### Example 3

In example 3 the experiment was performed similar to example 2 until step C).

In step C), the washed organic phase was concentrated to obtain the dry form of the concentrated organic phase having the product, 2-chloro-4-fluoro-N-[isopropyl(methyl)sulfamoyl]-5-nitrobenzamide (Ia) with UPLC purity of 95.4 %.

In sub-step C3), the dry form of concentrated organic phase was charged with methanol (127 mL) and heated to a temperature in the range of 60 to 65°C to obtain a solution of the concentrated organic phase in clear form. To the above solution water (381 mL) was charged while maintaining the temperature in the range of 75°C to 80°C for a period of 60 min.

In step D), the solution of step C) of the concentrated organic phase was gradually cooled to a temperature of 30°C to 35°C to obtain suspension.

In step E), the suspension was filtered, washed with water (60 mL) and then dried under vacuum at temperature of 70°C to obtain the product 2-chloro-4-fluoro-N-[isopropyl(methyl)sulfamoyl]-5-nitro-benzamide (Ia) (107 gm, 91.1 %) with UPLC purity of 98.445 %.

### Example 4

In example 4 the experiment was performed similar to example 2 until step C).

In step C), the washed organic phase was concentrated to obtain 59 % solution of the concentrated organic phase having 97.01 % of UPLC purity of 2-chloro-4-fluoro-N-[isopropyl(methyl)sulfamoyl]-5-nitro-benzamide (Ia).

In sub-step C4), the concentrated organic phase was heated to a temperature in the range of 75 to 80°C. n-Hexane (85 mL) was charged to above solution and maintained the temperature at 70 to 75°C for 60 min.

In step D), the solution of the concentrated organic phase was gradually cooled to a temperature in range of 0°C to 5°C to obtain suspension.

In step E), the suspension was filtered, washed with a mixture of n-Hexane and chlorobenzene(72 mL, 1:1) and then dried under vacuum at 70°C to obtain the product, 2-chloro-4-fluoro-N-[isopropyl(methyl)sulfamoyl]-5-nitro-benzamide(la)(108 gm, 92 %) with UPLC purity of98.93 %, (98.90 %w/w).

### Example 5

In example 5 the experiment was performed similar to example 2 until step C).

In step C), the washed organic phase was concentrated to obtain solid form of the concentrated organic phase having 97.20 % of UPLC purity of 2-chloro-4-fluoro-N-[isopropyl(methyl)sulfamoyl]-5-nitro-benzamide (Ia).

In sub-step C4), the concentrated organic phase was charged with chlorobenzene (85 mL) and heated to 75°C to 80°C. Additionally n-Hexane (85 mL) was charged while maintaining the temperature in range of 70°C to 75°C for 60 min.

In step D), the concentrated organic phase was cooled to a temperature in range of 0°C to 5°C to obtain a suspension.

In step E), the suspension was filtered, washed with a mixture of n-Hexane and chlorobenzene (72 mL 1:1) and dried under vacuum at 70°C to give the product 2-chloro-4-fluoro-N-[isopropyl(methyl)sulfamoyl]-5-nitro-benzamide(la)(108.9 gm , 92.8 %)with UPLC purity of 99.11 %, (98.99 %w/w).

### Example 6

In example 6 the experiment was performed similar to example 2 until step C).

In step C), the washed organic phase obtained in step B) was concentrated to obtain a solution having 50 % solution of the concentrated organic phase of the product having 96.817 % UPLC purity of 2-chloro-4-fluoro-N-[isopropyl(methyl)sulfamoyl]-5-nitro-benzamide (Ia) and 0.778 % of 2-chloro-4-fluoro-5-nitro-benzoic acid (II) as impurity.

In step C5), the solution of concentrated organic phase was charged with 200 mL water (200 mL) and heated to a temperature in the range of 80°C to 85°C. Chlorobenzene was removed by vacuum distillation while maintaining water (200 mL) quantity in the flask. After complete removal of chlorobenzene, methanol (150 mL) was added to the concentrated organic phase and maintained the above temperature for 30 min.

In step D), the concentrated organic phase was gradually cooled to a temperature in range of 0°C to 5°C to obtain a suspension.

In step E), the suspension was filtered, washed with water (50 mL) and dried under vacuum at 70°C to obtain the product 2-chloro-4-fluoro-N-[isopropyl(methyl)sulfamoyl]-5-nitro-benzamide(la)(109 gm,: 92.84 %) with UPLC Purity of 96 %, (83.83 % w/w).

### Example 7

In example 7 the experiment was performed similar to example 2 until step C).

In step C), the washed organic phase obtained in step B) was concentrated to obtain a solution having 69 % solution of the concentrated organic phase having 98.05 % of UPLC Purity of 2-chloro-4-fluoro-N-[isopropyl(methyl)sulfamoyl]-5-nitro-benzamide (Ia).

In sub-step C4), the above solution of the concentrated organic phase was heated to a temperature in range of 80°C to 85°C, maintained for 30 min. Charged n-Hexane (121.4 mL) to the above solution while maintaining the temperature for additional 30 min.

In step D), the above solution of the concentrated organic phase was gradually cooled to a temperature in range of 0°C to 5°C to obtain a suspension.

In step E), the suspension was filtered, washed with a mixture of chlorobenzene and n-Hexane (48 mL 1:1) and then dried under vacuum at 70°C to obtain the product 2-chloro-4-fluoro-N-[isopropyl(methyl)sulfamoyl]-5-nitro-benzamide(la) (109.7 gm, 93.48 %) with UPLC purity of98.8 %, (99.76 % w/w).

### Example 8

In example 8 the experiment was performed similar to example 1 until step C).

In step C), the washed organic phase obtained in step B) was concentrated to obtain a solution having 61 % of the product with UPLC Purity of 98.05 % of the 2-chloro-4-fluoro-N-[isopropyl(methyl)sulfamoyl]-5-nitro-benzamide (Ia).

In sub-step C4), the concentrated organic phase was heated to a temperature in the range of 80°C to 85°C and maintained for 30 min. Charged n-hexane (121.4 mL), while maintaining the above temperature for 30 min.

In step D), the concentrated organic phase was gradually cooled to a temperature in the range of 0°C to 5°C to obtain a suspension.

In step E), the suspension was filtered and dried under vacuum at 70°C to obtain the product, 2-chloro-4-fluoro-N-[isopropyl(methyl)sulfamoyl]-5-nitro-benzamide(la) (114.38 gm, 97.4 %) with UPLC purity of 98.1 %, (97.05 % w/w).

## Claims

1. A method of isolating a 3-nitrobenzenesulfonamide of the formula (I), 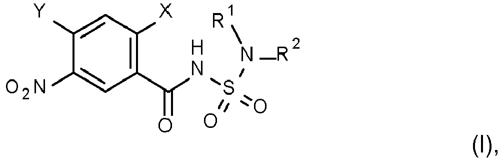 wherein
R¹ and R² are each independently selected from the group consisting of substituted or unsubstituted, linear or branched C₁-C₈-alkyl; substituted or unsubstituted C₃-C₈-cycloalkyl; or R¹ and R² together with the nitrogen atom to which they are bonded, form a 5-, 6-, 7- or 8-membered substituted or unsubstituted, saturated or unsaturated heterocyclic ring which, may have further 1 or 2 heteroatoms selected from O and S, and
X and Y are each independently selected from the group consisting of H, F, Cl, Br, I and CN,
from a reaction mixture comprising the 3-nitrobenzenesulfonamide of the formula I comprising at least the steps of
A. acidifying the reaction mixture with at least one acid to adjust the pH in the range of ≥ 3 to ≤ 6 and obtaining an organic phase and an aqueous phase;
B. separating the organic phase and washing the organic phase with at least one base to obtain a washed organic phase and a washed aqueous phase;
C. concentrating the washed organic phase obtained in step B);
D. cooling the concentrated organic phase to obtain a suspension, and
E. isolating the 3-nitrobenzenesulfonamide of the formula (I) from the suspension.

2. The method according to claim 1, wherein
R¹ is methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl or 1-ethylpropyl;
R² is methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl or 1-ethylpropyl
X is F or CI; and
Y is For Cl.

3. The method according to claim 1, wherein the reaction mixture is obtained by reacting a sulfuric diamide of the formula (i)
R¹R²N-S(O)₂-NH₂ (i)
in which R¹ and R² are each defined as in claim 1 or 2,
with a 3-nitrobenzoyl chloride of the formula (ii) 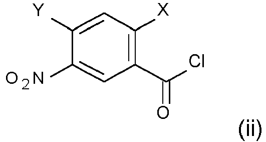 in which X and Y are each defined as in claim 1 or 2.

4. The method according to claim 3, wherein the reaction mixture is obtained by
(a) mixing the sulfuric diamide of the formula (i) with at least one aromatic solvent and at least one phase transfer catalyst; to obtain a mixed solution, and
(b) adding a base solution and 3-nitrobenzoyl chloride of the formula (ii) to the mixed solution of step (a) to form the reaction mixture.

5. The method according to claim 4, wherein the at least one aromatic solvent is selected from the group consisting of benzene, alkylbenzenes and halobenzene.

6. The method according to claim 4, wherein the temperature in step (a) is in the range of ≥ 10 °C to ≤ 15°C.

7. The method according to claim 4, wherein the base is selected from the group consisting of alkali metal and alkaline earth metal hydroxides.

8. The method according to claim 7, wherein the alkali metal and alkaline earth metal hydroxide is selected from the group consisting of lithium hydroxide, sodium hydroxide, magnesium hydroxide, potassium hydroxide, and calcium hydroxide.

9. The method according to claim 4, wherein the sulfuric diamide of the formula (i) is 2-[methyl(sulfamoyl)amino]propane.

10. The method according to claim 4, wherein the 3-nitrobenzoyl chloride of the formula (ii) is 2-chloro-4-fluoro-5-nitro-benzoyl chloride.

11. The method according to claim 4, wherein the 3-nitrobenzenesulfonamide of formula (I) is 2-chloro-4-fluoro-N-[isopropyl(methyl)sulfamoyl]-5-nitro-benzamide.

12. The method according to claim 1, wherein in step A) the at least one acid is selected from inorganic acid and organic acid.

13. The method according to claim 1, wherein the pH of step A) is in the range of ≥ 3 to ≤ 4.

14. The method according to claim 1, wherein the reaction mixture of step A) is maintained at a temperature in the range of 50°C to 80°C for a period of 5 to 30 min.

15. The method according to claim 1, wherein in step B) the at least one base in step B) is selected from the group consisting of sodium acetate, lithium acetate, potassium acetate. sodium carbonate and potassium carbonate.

16. The method according to claim 1, wherein the concentrated organic phase of step C) is treated by at least one-sub-step selected from the steps of:
C1) heating the concentrated organic phase of step C) to a temperature in the range of ≥ 50°C to ≤ 70°C for a period of ≥ 5 to ≤ 60 min,
C2) adding water to the concentrated organic phase of step C), heating to a temperature in the range of ≥ 60°C to ≤ 90°C, and concentrating,
C3) adding at least one alcohol to the concentrated organic phase of step C), heating to a temperature in the range of ≥ 50°C to ≤ 90°C, adding water, and heating to a temperature in the range of ≥ 70°C to ≤ 90°C,
C4) heating the concentrated organic phase to a temperature in the range of ≥ 70°C to ≤ 90°C, adding at least one hydrophobic solvent to the concentrated organic phase of step C) and maintaining at a temperature in the range of ≥ 70°C to ≤ 90°C for a period of ≥ 5 to ≤ 120 min, and
C5) adding water to the concentrated organic phase of step C), heating to a temperature in the range of ≥ 60°C to ≤ 90°C, concentrating, and adding at least one alcohol.
